(19)

**Europäisches Patentamt**
**European Patent Office**
**Office européen des brevets**

(11) **EP 3 731 912 B1**

(12) **EUROPEAN PATENT SPECIFICATION**

(45) Date of publication and mention
of the grant of the patent:
**27.04.2022 Bulletin 2022/17**

(21) Application number: **18827101.9**

(22) Date of filing: **21.12.2018**

(51) International Patent Classification (IPC):
$A61M\ 16/16^{(2006.01)}$ $G16H\ 20/40^{(2018.01)}$
$G16H\ 40/63^{(2018.01)}$ $A61M\ 16/06^{(2006.01)}$
$A61M\ 16/00^{(2006.01)}$ $A61M\ 16/20^{(2006.01)}$

(52) Cooperative Patent Classification (CPC):
**A61M 16/0666; A61M 16/205; G16H 20/40;**
**G16H 40/63;** A61M 16/026; A61M 16/16;
A61M 16/204; A61M 2016/0027; A61M 2016/0039;
A61M 2205/15; A61M 2205/18; A61M 2205/3553;
A61M 2205/3561; A61M 2205/52; A61M 2205/581

(86) International application number:
**PCT/EP2018/086458**

(87) International publication number:
**WO 2019/129676 (04.07.2019 Gazette 2019/27)**

(54) **SYSTEM FOR PROVIDING NASAL THERAPY**

SYSTEM ZUR BEREITSTELLUNG EINER NASENTHERAPIE

SYSTÈME POUR FOURNIR UNE THÉRAPIE NASALE

(84) Designated Contracting States:
**AL AT BE BG CH CY CZ DE DK EE ES FI FR GB**
**GR HR HU IE IS IT LI LT LU LV MC MK MT NL NO**
**PL PT RO RS SE SI SK SM TR**

(30) Priority: **28.12.2017 US 201762611167 P**

(43) Date of publication of application:
**04.11.2020 Bulletin 2020/45**

(73) Proprietor: **Koninklijke Philips N.V.**
**5656 AG Eindhoven (NL)**

(72) Inventors:
• **TRUSCHEL, William, Anthony**
  **5656 AE Eindhoven (NL)**

• **HETE, Bernard, F**
  **5656 AE Eindhoven (NL)**

(74) Representative: **Philips Intellectual Property &**
**Standards**
**High Tech Campus 52**
**5656 AG Eindhoven (NL)**

(56) References cited:
**EP-A2- 0 691 134** **WO-A1-02/28460**
**WO-A1-2010/076712** **WO-A1-2011/141845**
**WO-A1-2011/145014** **US-A- 5 865 173**

**Description**

BACKGROUND OF THE INVENTION

1. Field of the Invention

The present disclosure pertains to a system for providing high-flow nasal therapy to a patient.

2. Description of the Related Art

Invasive therapy is a means for providing breathing therapy to a patient. With invasive therapies, the breathing therapy is delivered to the patient using an artificial tube, such as an endotracheal tube or a tracheotomy tube. Stepping down from that is non-invasive ventilation ("NIV"). With invasive therapies, the breathing therapy is often provided temporarily to the patient in a hospital or hospital-like environment, where the patient may be monitored continually, and adjustments to the therapy may be performed by a doctor, nurse, and/or other skilled clinician. With NIV, a patient interface, such as a non-rebreathing mask or nasal cannula, a machine (e.g., a ventilator) delivers therapy to a patient. NIV can be used in the hospital as a temporary remedy for acute respiratory symptoms and/or as a long-term therapy for patients that require care in the home due to a variety of issues (e.g., chronic lung disease, airway abnormality, and/or a neuromuscular syndrome).

The following documents EP 0 691 134, WO 02/28460, WO 2010/076712, WO 2011/141845, US 5 865 173 and WO 2011/145014 also disclose systems for providing high-flow nasal therapy comprising controlled inhalation and exhalation valves.

SUMMARY OF THE INVENTION

The invention is defined in independent claim 1. The dependent claims describe preferred embodiments of the invention. Accordingly, one or more aspects of the present disclosure relates to a nasal therapy system. The nasal therapy system includes a nasal interface, at least one flow sensor, at least one pressure sensor, a valve, and a controller. The at least one flow sensor is configured to determine a flow rate of a gas flow to be provided to a patient via the nasal interface. The at least one pressure sensor is configured to determine an amount of pressure of the gas flow based, at least in part, on a prescribed pressure. The valve is in communication with the nasal interface and is operable to control an amount of the gas flow being provided to the nasal interface. The controller is operatively coupled to the at least one flow sensor, the at least one pressure sensor, and the valve. The controller is operatively coupled to the at least one flow sensor, the at least one pressure sensor, and the valve. The controller is configured to determine a flow rate difference between a prescribed flow rate and the flow rate, and cause an output setting of the valve to be adjusted to control the amount of the gas flow being provided to the nasal interface such that the amount of the gas flow being provided to the nasal interface is maintained at the prescribed flow rate and the prescribed pressure, where the output setting is determined based on the flow rate difference.

Another aspect of the present disclosure relates to a method for providing nasal therapy. The method includes determining a flow rate of a gas flow to be provided to a patient via a nasal interface. An amount of pressure of the gas flow is determined based, at least in part, on a prescribed pressure. A controller, operatively coupled to a valve, the valve being in communication with the nasal interface, determines a flow rate difference between a prescribed flow rate and the flow rate, the valve being operable to control an amount of the gas flow being provided to the nasal interface. An output setting of the valve is caused to be adjusted to control the amount of the gas flow being provided to the nasal interface such that the amount of the gas flow provided to the nasal interface is maintained at the prescribed flow rate and the prescribed pressure, where the output setting is determined based on the flow rate difference.

Yet another aspect of the present disclosure relates to a nasal therapy system. The nasal therapy system includes interfacing means, first sensing means, second sensing means, venting means, and controlling means. The first sensing means are configured to determine a flow rate of a gas flow to be provided to a patient via the interfacing means. The second sensing means are configured to determine an amount of pressure of the gas flow based, at least in part, on a prescribed pressure. The venting means are in communication with the interfacing means and are operable to control an amount of the gas flow being provided to the interfacing means. The controlling means are operatively coupled to the first sensing means, the second sensing means, and the venting means, where the controlling means are configured to determine a flow rate difference between a prescribed flow rate and the flow rate, and cause an output setting of the venting means to be adjusted to control the amount of the gas flow being provided to the interfacing means such that the amount of the gas flow provided to the interfacing means is maintained at the prescribed flow rate and the prescribed pressure, where the output setting is determined based on the flow rate difference.

These and other objects, features, and characteristics of the present disclosure, as well as the methods of operation and functions of the related elements of structure and the combination of parts and economies of manufacture, will become more apparent upon consideration of the following description and the appended claims with reference to the accompanying drawings, all of which form a part of this specification, wherein like reference numerals designate corresponding parts in the various figures. It is to be expressly understood, however, that the drawings are for the purpose of illustration and description only and are not intended as a definition of the limits of the disclosure.

BRIEF DESCRIPTION OF THE DRAWINGS

FIG. 1A is an illustrative diagram of an exemplary system configured to provide high-flow nasal therapy to a patient, in accordance with various examples;
FIG. 1B is an illustrative diagram of the controller of FIG. 1A, in accordance with various examples;
FIG. 1C is an illustrative schematic of the exemplary system of FIG. 1A, in accordance with the invention;
FIG. 2A is a schematic illustration of the system of FIG. 1A and 1C;
FIG. 2B is an illustrative schematic of a portion of system 100 that facilitates pressure control in accordance with various embodiments;
FIG. 3 is an illustrative diagram of a patient interface used within the exemplary system of FIG. 1A, in accordance with various embodiments;
FIGS. 4A-E are illustrative graphs describing exemplary pressure measurements, flow rates, patient flow rates, current values, and breathing rates, in accordance with various embodiments;
FIG. 5 is an illustrative flowchart of an exemplary process for adjusting a valve, in accordance with various embodiments;
FIGS. 6A and 6B are illustrative diagrams of a side view and perspective view of an exemplary flow controller, in accordance with various embodiments;
FIG. 7 is an illustrative diagram of a patient interface including a nasal interface, in accordance with various embodiments;
FIGS. 8A-C are an illustrative graphs of a detected high leak scenario, in accordance with various embodiments;
FIGS. 9A-C are illustrative graphs of a no patient detected scenario, in accordance with various embodiments; and
FIG. 10 is an illustrative diagram of an alert system and monitor in communication with a controller, such as that of FIGS 1A-C, in accordance with various examples.

DETAILED DESCRIPTION OF EXEMPLARY EMBODIMENTS

As used herein, the singular form of "a", "an", and "the" include plural references unless the context clearly dictates otherwise. As used herein, the term "or" means "and/or" unless the context clearly dictates otherwise. As used herein, the statement that two or more parts or components are "coupled" shall mean that the parts are joined or operate together either directly or indirectly, i.e., through one or more intermediate parts or components, so long as a link occurs. As used herein, "directly coupled" means that two elements are directly in contact with each other. As used herein, "fixedly coupled" or "fixed" means that two components are coupled so as to move as one while maintaining a constant orientation relative to each other. As used herein, "fluidly coupled," "fluidly connected," "in fluid communication," or "in gas communication" means that two or more components are connected together such that fluid, gas, and/or a mixture of fluid and gas are capable of passing between the two or more components with minimal to no leak to an ambient environment (e.g., less than 5% leak).

As used herein, the word "unitary" means a component is created as a single piece or unit. That is, a component that includes pieces that are created separately and then coupled together as a unit is not a "unitary" component or body. As employed herein, the term "number" shall mean one or an integer greater than one (i.e., a plurality).

Directional phrases used herein, such as, for example and without limitation, top, bottom, left, right, upper, lower, front, back, and derivatives thereof, relate to the orientation of the elements shown in the drawings and are not limiting upon the claims unless expressly recited therein.

High-flow nasal therapy, generally speaking, can provide numerous advantages for patients. For instance, high-flow nasal therapy facilitates the clearing of $CO_2$ gas from the upper airway during expiration. Additionally, high-flow nasal therapy provides pressure during inspiration, thereby allowing for improved gas exchange. For example, an increased partial pressure of oxygen in a patient's airways at the alveolar level increases transmission of oxygen from the lungs into the blood. Another advantage of high-flow nasal therapy is that high-flow nasal therapy is typically comfortable for patients. Most non-invasive ventilation techniques typically involves a mask that covers up most of the patient's face, thereby limiting the patient's ability to carry a conversation, eat, or do many other activities due to the patient's face being covered by the mask. Additionally, non-invasive ventilation therapies, partially due to the mask, may cause the patient to feel claustrophobic, and may be overbearing. In contrast, high-flow nasal therapy may mitigate many of the adverse effects of other non-invasive therapies because high-flow nasal therapy involves nasal prongs residing on a patient's upper lip.

While some prior therapy products are capable of providing therapy at a high-flow setting, these products only allow for a flow rate and/or oxygen concertation (e.g., between 21% and 100%) to be set. These therapy products do not provide any monitoring of the patient or nasal therapy to the patient and/or clinician. Further still, while some pressure support may be available (e.g., continuous positive airway pressure ("CPAP")), that pressure support is often uncontrolled.

The high-flow nasal therapy system described herein may allow, amongst other aspects, a caregiver to set a pressure setting for the high-flow nasal therapy, as well as patient flow monitoring. Furthermore, the systems and techniques described herein allow for a patient's breathing performance to be tracked. For example, the high-flow nasal therapy system may be capable of measuring breath rate, determining when a patient has become disconnected from the system, and measuring possible tidal volume of inspiration and expiration. Furthermore, in addition to the above-mentioned advantages, the high-flow nasal therapy system described herein is capable of providing high-flow nasal therapy with blended oxygen and washing out $CO_2$ from the patient's airway, while still being comfortable and unencumbering to the patient.

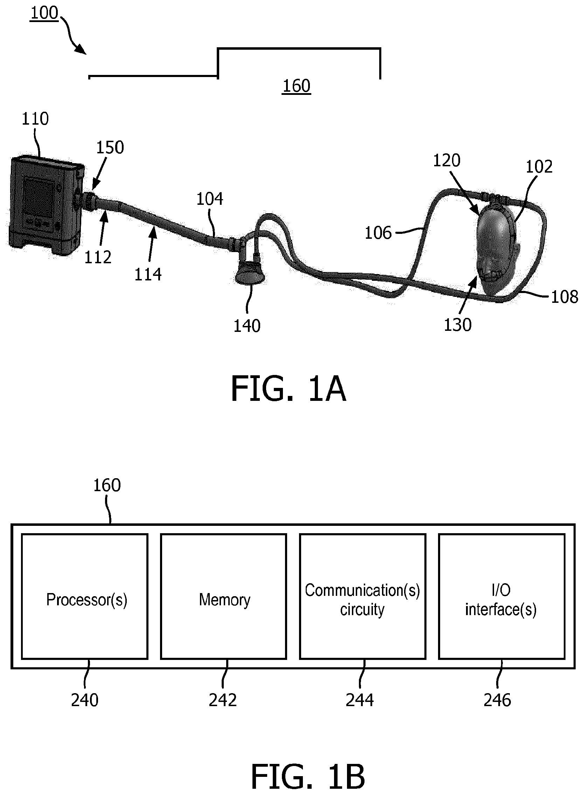

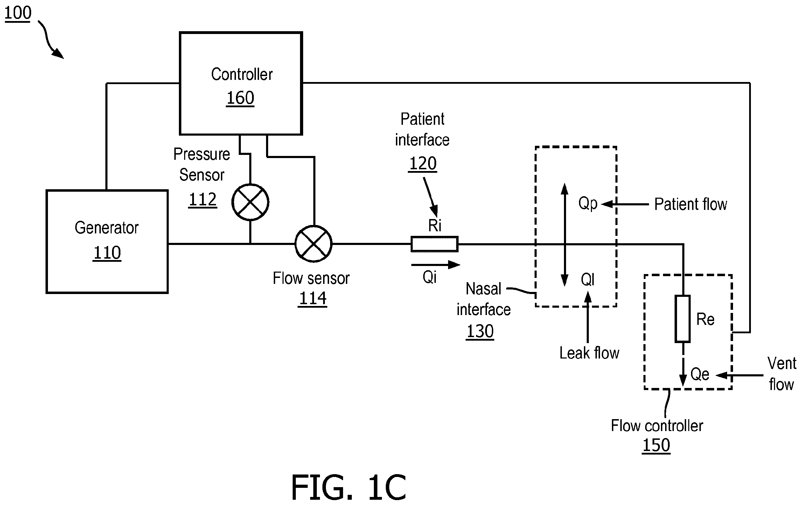

FIG. 1 is an illustrative diagram of an exemplary system 100 configured to provide high-flow nasal therapy to a patient, in accordance with various embodiments. In one embodiment, nasal therapy system 100 is a high-flow nasal therapy system configured to provide high-flow nasal therapy to a patient. High-flow nasal therapy, may be characterized as any gas flow prescribed to a patient via the patient's nasal airway that exceeds approximately 15 L/min (liters per minute). In one embodiment, high-flow nasal therapy is combined with oxygen therapy. For example, high-flow nasal therapy may correspond to an oxygen therapy provided to a patient at approximately 20 L/min, 30 L/min, 40 L/min, 50 L/min, 60 L/min, etc. The combination of high-flow nasal therapy and oxygen therapy may be referred to as high-flow oxygen therapy. As an illustrative example, a patient suffering from acute exasperation of chronic obstructive pulmonary disease ("COPD") or hypoxemia may be prescribed high-flow nasal therapy over standard non-invasive ventilation.

Nasal therapy system 100, in one non-limiting embodiment, corresponds to a high-flow nasal therapy system capable of providing high-flow nasal therapy to a patient 102. Nasal therapy system 100, in the illustrative embodiment, includes a gas generator 110, a pressure sensor 112, a flow sensor 114, a humidifier 140, a patient interface 120, and a controller 160. Furthermore, patient interface 120 includes a nasal interface 130.

Gas generator 110, also referred to herein as generator 110 or pressure generator 110, in one embodiment, is configured to generate a pressured gas flow deliverable to a patient. For example, generator 110 may correspond to a ventilator and/or pump that generates a gas flow (e.g., oxygen gas) for use in a breathing therapy provided to a patient. In the illustrative embodiment, generator 110 is fluidly connected to humidifier 140 via a first gas line 104. For example, first gas line 104 may be a tubing or other fluid/gas communications means. Generator 110 is capable, for instance, of producing and outputting high-flow gas, which may or may not include oxygen-enriched gas. For example, generator 110, as described in greater detail below, may include a pump, also referred to herein as a fan, which may receive air from an ambient environment, and causes the air to be output from generator 110 at a particular flow rate and/or pressure (e.g., 5 cm of water pressure ("cmH20")).

In one embodiment, generator 110 includes, or is otherwise coupled to, a gas source, such as, and without limitation, an oxygen source. In this particular scenario, where generator 110 includes or is otherwise coupled to a gas source, generator 110 is capable of outputting high-flow gas blended with gas received from the gas source. For example, if the gas source is an oxygen tank, or oxygen reservoir, then the high-flow gas output by generator 110 may be of a particular oxygen concentration level (e.g., 21% oxygen concentration to 100% oxygen concentration). As another example, the gas flow may have a flow rate of 15 L/min or greater. The gas source is capable of being portable in one embodiment. For example, gas source may be a portable oxygen tank, thereby enabling system 100 to be mobile.

First gas line 104 is, as mentioned above, capable of receiving high-flow gas output by generator 110, and providing that high-flow gas to humidifier 140. Humidifier 140, in one embodiment, is configured to heat the high-flow gas to a prescribed level such that the high-flow gas provided to patient 102 is warmed and humidified. For flow-rates in the range of 50-60 L/min, for example, high levels of humidity may be needed for the delivered therapy. This may be due to the mucus membranes in the lungs and other portions of the airways drying out as a result of the flow rate associated with high-flow therapy, and therefore an external mechanism (e.g., humidifier 140) is needed to humidify the gas (e.g., air). Humidifier 140 may correspond to any device that is capable of heating the gas output by generator 110. For example, humidifier 140 may include a heater plate that heats to a particular temperature, thereby heating water molecules in the gas to humidify that high flow gas. In particular, the humidity for non-invasive therapy, such as high-flow nasal therapy, should be on par with the humidity used when applying an invasive therapy. Persons of ordinary skill in the art will recognize that, in some embodiments, generator 110 may include humidifier 140 therein, or may include heating means, and the use of humidifier 140 within system 100 is merely exemplary.

Patient interface 120, in one embodiment, is in fluid communication with heater 140 via a second channel 106 and a third channel 108. Each of channels 106 and 108 are configured to deliver the heated and humidified high-flow gas from humidifier 140 to patient interface 120. In one embodiment, only one of channels 106 and 108 deliver the heated and humidified high-flow gas to patient interface 120, while the other of channels 106 and 108 may be configured to receive and/or facilitate venting of exhaled gas/air from patient 102. Furthermore, in one embodiment, system 100 includes only a single channel (e.g., one of channels 106 and 108) as opposed to two channels.

As described in greater detail below, patient interface 120 includes nasal interface 130. Nasal interface 130 is configured to provide the gas flow to a patient's airway via one or more gas outlets. In one embodiment, patient interface 120 includes, or is otherwise coupled with, one or more sensors configured to measure a flow rate and/or a pressure of the gas flow being provided to the patient via nasal interface 130. For instance, nasal interface 130 includes a flow rate sensor in communication with a gas outlet of the nasal interface such that a flow rate and/or a pressure of the gas flow at the gas outlet is capable of being determined.

Pressure sensor 112 is configured, in one embodiment, to measure a pressure of the gas flow output by generator 110. Pressure sensor 112 is located at an outlet of generator 110 and is further in communication with controller 160. The measured pressure is related to a prescribed pressure (e.g., a desired pressure of the breathing therapy), which may be input into controller 160. The measured pressure is further related to a resistance, also referred to as a "pressure drop," along tubing 104, and the flow rate measured by flow sensor 114. The resistance, in one embodiment, is a known value such that the pressure drop to the nasal interface is known (e.g., 5-10 cmH20 drop from generator 110 to nasal interface 130).

Flow sensor 114 is configured to measure a flow rate of the gas flow output to nasal interface 130. In one embodiment, flow sensor 114 is located at the outlet of generator 110 proximate to pressure sensor 112, however this is merely illustrative. Flow sensor 114 is capable of determining the flow rate (e.g., flow rate $Q_i$) of the gas flow and a difference between a prescribed flow rate and the flow rate. The flow rate $Q_i$, in one embodiment, corresponds to a sum of the gas flow output from system 100. For instance, a portion of the gas flow is output to patient 102, a portion of the gas flow may be output via a leak existing between nasal interface 130 and patient 102, and a portion of the gas flow is capable of being vented to an ambient environment. Flow sensor 114 is configured to measure the flow rate and determine whether the flow rate differs from the prescribed flow rate (e.g., 1 liter per second ("lps")).

System 100 also, in one embodiment, includes a flow controller 150. According to an embodiment the flow controller 150 is operable to adjust an amount of the gas flow capable of being vented to an ambient environment. In one embodiment, a second flow control is also included within system 100 that is capable of controlling the gas flow output by generator 110 to channel 104. Flow controller 150 is capable of causing a flow rate of the gas flow to be adjusted according to a particular flow rate setting prescribed for the patient. For instance, flow controller 150 is configured to adjust an output setting to maintain a constant flow rate, such as a prescribed flow rate, and pressure, such as a prescribed pressure, of gas flow provided to patient 102. In one embodiment, flow controller 150 is a throttle valve, which may be mechanical, electrical, and/or a combination of mechanical and electrical. In the illustrative embodiment, flow controller 150 is located at an outlet of generator 110 such that generator 110 is in fluid communication with channel 104 via flow controller 150. Flow controller 150, according to the invention, includes an adjustable valve operable to cause an output setting of the gas flow to be modified. In this scenario, flow controller 150 is configured to modify an amount of gas flow capable of exiting a gas outlet of nasal interface 130 such that a pressure and/or flow rate of the gas flow output to patient 102 via one or more nasal outlets of nasal interface 130 is adjustable. While flow controller 150, in one embodiment, is located at an outlet of generator 110, alternatively (or additionally) flow controller 150 is capable of being placed at an exhaust of nasal interface 130.

Controller 160 is configured, in one embodiment, to adjust an output setting of flow controller 150 to ensure that a pressure of the gas flow and the flow rate of the gas flow provided to patient 102 remains constant. In one embodiment, controller 160 is configured to adjust a pressure of the gas flow output by generator 110. To do this, controller 160 generates and sends control signals to generator 110, which causes the pressure of the output gas flow from generator 110 to change. For instance, controller 160 generates an instruction that is sent to gas generator 110, which in turn causes gas generator 110 to increase or decrease a pressure of the output gas flow to restore the pressure at nasal interface 130 to the prescribed pressure. According to the invention, controller 160 is coupled to pressure sensor 112 and flow sensor 114. In this particular scenario, controller 160 receives output signals from pressure sensor 112 and flow sensor 114, and in turn, processes the output signals to determine whether an adjustment is needed to ensure that the gas flow output to patient 102 is at the prescribed pressure and flow rate.

Controller 160 is further configured to monitor one or more breathing characteristics of patient 102 by adjustments made to flow controller 150. For instance, when patient 102 inhales, an amount of the gas flow output to the pressure increases. In response, controller 160 determines that flow controller 150 will need to close (or approximately close), and will generate a signal to cause flow controller 150 to restrict the amount of gas flow exiting therefrom. The signal (e.g., a current provided to the valve) will therefore modulate based on the patient's breathing pattern. Controller 160 is therefore capable of determining, amongst other features, a breathing rate of patient 102, a tidal volume of breathing of patient 102, whether there is a substantial leak at nasal interface 130, and/or if patient 102 becomes disconnected or otherwise unable to receive the breathing therapy, all based on the detected changes provided to flow controller 150 from controller 150.

FIG. 1B is an illustrative diagram of the controller of FIG. 1A, in accordance with various embodiments. Controller 160, in the illustrative embodiment, includes one or more processors 240, memory 242, communications circuitry 244, and I/O interface 246. Controller 160, in one embodiment, corresponds to a user device. A user device, for example, may correspond to any suitable type of electronic device including, but are not limited to, desktop computers, mobile computers (e.g., laptops, ultrabooks, etc.), mobile phones, smart phones, tablets, personal digital assistants ("PDAs"), and/or wearable devices (e.g., watches, pins/broaches, headphones, etc.).

Processor(s) 240 include any suitable processing circuitry capable of controlling operations and functionality of controller 160, as well as facilitating communications between various components within generator 160. In one embodiment, processor(s) 240 may include a central processing unit ("CPU"), a graphic processing unit ("GPU"), one or more microprocessors, a digital signal processor, or any other type of processor, or any combination thereof. In another embodiment, the functionality of processor(s) 240 is performed by one or more hardware logic components including, but not limited to, field-programmable gate arrays ("FPGA"), application specific integrated circuits ("ASICs"), application-specific standard products ("ASSPs"), system-on-chip systems ("SOCs"), and/or complex programmable logic devices ("CPLDs"). Furthermore, each of processor(s) 240 is capable of including its own local memory, which may store program systems, program data, and/or one or more operating systems. However, processor(s) 240 are capable of running an operating system ("OS") for the user device and/or generator 110, and/or one or more firmware applications, media applications, and/or applications resident thereon. In one example embodiment, processor(s) 240 runs a local client script for reading and rendering content received from one or more websites.

Memory 242, in one embodiment, includes one or more types of storage mediums such as any volatile or non-volatile memory, or any removable or non-removable memory implemented in any suitable manner to store data for controller 160. For example, information may be stored using computer-readable instructions, data structures, and/or program systems. Various types of storage/memory may include, but are not limited to, hard drives, solid state drives, flash memory, permanent memory (e.g., ROM), electronically erasable programmable read-only memory ("EEPROM"), CD-ROM, digital versatile disk ("DVD") or other optical storage medium, magnetic cassettes, magnetic tape, magnetic disk storage or other magnetic storage devices, RAID storage systems, or any other storage type, or any combination thereof. Furthermore, memory 242 may be implemented as computer-readable storage media ("CRSM"), which may be any available physical media accessible by processor(s) 240 to execute one or more instructions stored within memory 242. In an example embodiment, one or more applications (e.g., gaming, music, video, calendars, lists, etc.) are run by processor(s) 240, and may be stored in memory 242.

Communications circuitry 244, in one embodiment, corresponds to any circuitry allowing or enabling one or more components of controller 160 to communicate with one another, and/or with one or more additional devices, servers, and/or systems. As an illustrative example, data corresponding to readings obtained by a sensor in communication with nasal interface 130 may be transmitted over a network, such as the Internet, to controller 160 and/or a user device via communications circuitry 244 using any number of communications protocols. For example, networks are capable of being accessed via communications circuitry 244 using Transfer Control Protocol and Internet Protocol ("TCP/IP") (e.g., any of the protocols used in each of the TCP/IP layers), Hypertext Transfer Protocol ("HTTP"), WebRTC, SIP, and/or wireless application protocol ("WAP"). Various additional communication protocols used to facilitate communications by communications circuitry 244 include, but not limited to, Wi-Fi (e.g., 802.11 protocol), Bluetooth, radio frequency systems (e.g., 900 MHz, 1.4 GHz, and 5.6 GHz communication systems), cellular networks (e.g., GSM, AMPS, GPRS, CDMA, EV-DO, EDGE, 3GSM, DECT, IS-136/TDMA, iDen, LTE or any other suitable cellular network protocol), infrared, BitTorrent, FTP, RTP, RTSP, SSH, and/or VOIP.

In one embodiment, controller 160 includes one or more antennas to facilitate wireless communications with a network using various wireless technologies (e.g., Wi-Fi, Bluetooth, radiofrequency, etc.). In yet another embodiment, controller 160 includes one or more universal serial bus ("USB") ports, one or more Ethernet or broadband ports, and/or any other type of hardwire access port so that communications circuitry 244 is capable of communicating with one or more communications networks.

I/O interface(s) 246 is capable of corresponding to any suitable input and/or output component such as, and without limitation, one or more microphones or other audio input devices, one or more speakers or other audio output devices, one or more input mechanisms (e.g., buttons, knobs, switches, etc.), one or more cameras or other image capture devices, and/or one or more display screens. For example, controller 160 and/or a user device in communication with controller 160 may include a display screen of any suitable size and/or shape. Various types of displays include, but are not limited to, liquid crystal displays ("LCD"), monochrome displays, color graphics adapter ("CGA") displays, enhanced graphics adapter ("EGA") displays, variable graphics array ("VGA") display, or any other type of display, or any combination thereof. I/O interface 246 further is capable of including a touch screen, such as a touch screen including capacitive sensing panels capable of recognizing touch inputs thereon. For instance, a touch screen may correspond to a projected capacitive touch ("PCT"), screen include one or more row traces and/or driving line traces, as well as one or more column traces and/or sensing lines.

FIG. 1C is an illustrative schematic of the exemplary system of FIG. 1A, in accordance with various embodiments. Nasal therapy system 100, as seen in the illustrative embodiment of FIG. 1C, includes pressure generator 110, patient interface 120, nasal interface 130, flow controller 150, and controller 160. Also detailed within FIG. 1C are pressure sensor 112 and flow sensor 114. Although only a single instance of pressure sensor 112 and flow sensor 114 are shown within FIG. 1C, persons of ordinary skill in the art will recognize that multiple instances of pressure sensor 112 and/or flow sensor 114 may also be included.

In one example embodiment, a user, such as a patient, clinician, and/or physician, inputs a prescribed pressure and a prescribed flow rate for a breathing therapy to be provided to a patient (e.g., patient 102). For example, the prescribed pressure may be 10 cmH20 and the prescribed flow rate may be 60 lpm. As seen within FIG. 1C, resistance Ri may correspond to an amount of pressure "loss" between the gas flow output by generator 110 and nasal interface 130. This resistance (e.g., resistance Ri) is known, in one embodiment, to controller 160 prior to the breathing therapy being provided to the patient. For example, resistance Ri may be determined to be 10 cmH20/lps, meaning that there is a pressure drop of 10 cmH20 per liter per second ("lps"). In other words, if the desired pressure at nasal interface 130 is 10 cmH20 and there is a 10 cmH20 drop between generator 110 and nasal interface 130 (e.g., along tube 104 and/or humidifier 140), then generator 110 should output a gas flow having a pressure of 20 cmH20.

In FIG. 1C, Qi corresponds to the gas flow that is provided to nasal interface 130. Controller 160 is operable, in one embodiment, to maintain Qi to be the prescribed flow rate. At nasal interface 130, gas flow is capable of being output via three different mechanisms. The first is patient flow, represented by Qp - corresponding to an amount of the gas flow that is inhaled by the patient. The second is leak flow, represented by Ql - corresponding to an amount of the gas flow lost due to a leak between the patient and nasal interface 130. The third is vent flow, represented by Qe - corresponding to an amount of the gas flow that is vented to an ambient environment. Resistance Re relates to a setting of flow controller 150 that controls an amount of Qe. For example, increasing Re may cause flow controller 150 (e.g., a valve) to restrict the value of Qe, while decreasing Re may cause flow controller 150 to increase the valve of Qe.

Controller 160, as mentioned above, operates to control the value of Qi by modulating the value of Re, hence the valve of Qe. Flow sensor 114 is capable of measuring Qi and providing data representing a measured flow rate to controller 160. Controller 160 employs Equation 1, in an example embodiment:

$$Qi = Qp + Ql + Qe \qquad \text{Equation 1.}$$

Using Equation 1, if the measured value of Qi is less than the prescribed flow rate, then controller 160 generates a signal to increase an amount of current provided to flow controller 150 to allow an increase in the amount of gas vented (e.g., increase Qe). Conversely, if the measured value of Qi is greater than the prescribed flow rate, then controller 160 generates a signal to decrease an amount of current provided to flow controller 150 to allow for a decrease in the amount of gas vented (e.g., decrease Qe). Persons of ordinary skill in the art will recognize that, alternatively, an increase in the current to flow controller 150 may decrease the amount of gas vented, while decreasing the current to flow controller 150 may increase the amount of gas vented, and the specific configurations of flow controller 150 may vary.

With each breath that the patient takes, the value of Qp will increase, and with each exhalation of the patient, the value of Qp will decrease. Therefore, this change in flow to the patient causes a change in the measured flow rate Qi, which in turn causes controller 160 to module the current provided to flow controller 150. In the illustrative embodiment, controller 160 operates to keep Qi at, or approximately, at, the prescribed flow rate during the course of the patient's breathing.

The pressure measured by pressure sensor 112, in one embodiment, is related to the measured flow rate Qi by Equation 2:

$$P\_measured = P\_prescribed + Qi * Ri \qquad \text{Equation 2.}$$

Based on Equation 2, the amount of pressure that is measured by pressure sensor 112 is determined by computing the sum of the prescribed pressure with the product of the measured flow rate Qi and the known resistance Ri. In one embodiment, pressure sensor 112 is configured to send data representing a measured pressure to controller 160. As controller 160 knows the desired pressure (e.g., prescribed pressure) to be received by the patient for the breathing therapy, and also knows the pressure drop (e.g., Qi * Ri), controller 160 is operable to generate output signals to cause generator 110 to increase or decrease a pressure of the output gas flow to produce the described therapy.

As an illustrative example, if the prescribed flow rate is 60 lpm, or 1 lps, the prescribed pressure is 10 cmH20, and the known resistance Ri is 10 cmH20/lps, then, using Equation 2, for a measured flow rate Qi equal to 1 lps, the pressure measured by pressure sensor 112 would be 10 cmH20 + (10 cmH20/lps * 1 lps) = 20 cmH20. This would be mean that controller 160 would need to instruct generator 110 to generate a pressure for the output gas flow to be 20 cmH20. When the measured flow rate Qi changes, controller 160 operates to restore the flow rate to the prescribed flow rate by causing flow controller 150 (e.g., a valve) to increase or decrease the amount of gas vented (e.g., Qe). Similarly, controller 160 instructs generator 110 to increase or decrease a pressure of the output gas flow (e.g., by causing a fan generating the flow) in order to maintain the prescribed pressure at nasal interface 130.

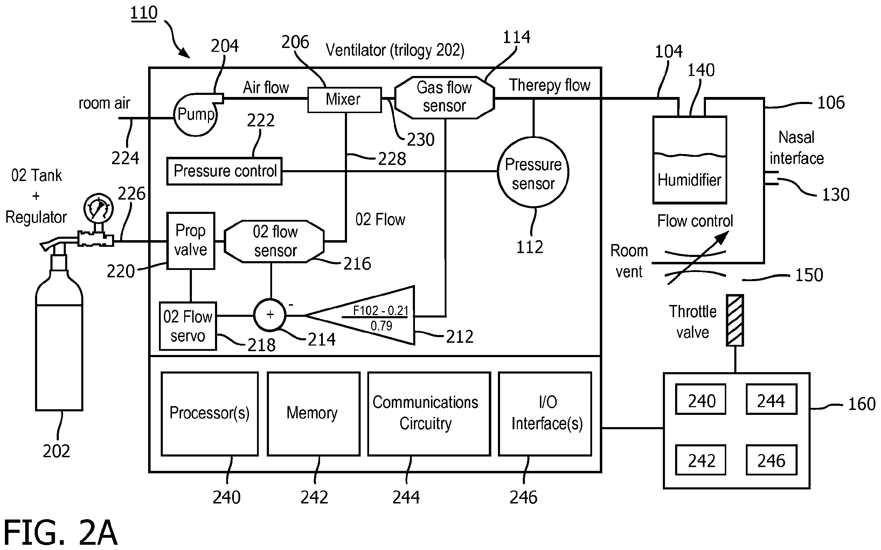

FIG. 2A is a schematic illustration of the system of FIG. 1A and 1C, in accordance with various embodiments. As mentioned previously with reference to FIG. 1A, high-flow nasal therapy system 100, in one embodiment, includes generator 110, humidifier 140, patient interface 120, flow controller 150, and a controller 160, where patient interface 120 further includes nasal interface 130. Not all of the components seen in FIGS. 1A and/or 1C are displayed within FIG. 2A, and this is simply for illustrative purposes and is not intended to be limiting. Similarly, not all of the components of FIG. 2A are displayed within FIGS. 1A and/or 1C, and this is simply for illustrative purposes and is not intended to be limiting.

In the illustrative embodiment, a gas source 202 is fluidly connected to generator 110 via a channel 226. Gas source 202, for example, may correspond to an oxygen tank (e.g., a portable oxygen tank or a non-portable oxygen tank), an oxygen reservoir, or any other type of oxygen source. Further still, gas source 202 may be referring to any suitable type of gas, and the use of oxygen is merely exemplary.

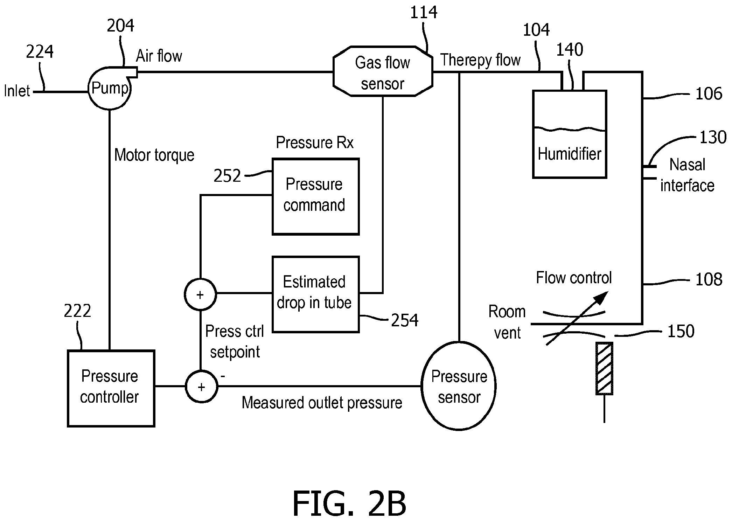

FIG. 2B is an illustrative schematic of a portion of system 100 that facilitates pressure control in accordance with various embodiments. Generator 110 is configured to receive ambient air via an inlet 224, which receives the ambient air and provides the ambient air to a pump 204. Pump 204, in one embodiment, is configured to pull ambient air in from an ambient environment external to generator 110, and cause the ambient air to be pressurized. Pump 204, in one embodiment, corresponds to a centrifugal pump or a blower. For example, the blower may be capable of generating and controlling pressure by modulating a rotary speed of a fan (e.g., fan blades). In one embodiment, pump 204 is connected to a pressure controller 222 that operates to control an amount of pressure of the air to be output by pump 204. The desired pressure setting is capable of being input by a patient (e.g., patient 102), a clinician (e.g., a nurse, physician's assistant, respiratory therapist, doctor), and/or by automatically through system 100 (e.g., via predefined settings, settings determined via machine-learning processing, etc.). For instance, a pressure command module 252 is capable of receiving an input pressure command from controller 160 (as seen in FIG. 1B), and indicating a desired pressure with which the therapy is to be delivered to the patient. The input pressure command, for example, refers to the amount of pressure of the gas flow that will be delivered to the patient at interface 130.

In one embodiment, the amount of pressure needed to be output by generator 110 is dependent on other characteristics of system 100. For example, there may be a pressure drop estimated based on, amongst other aspects, pressure loss within channel 104, humidifier 140, and channel 106 in fluid communication with interface 130 (as well as at other points within system 100). An estimated pressure drop module 254 is configured to estimate a pressure drop based on the characteristics of the system as a function of flow within system 100. In one embodiment, the pressure drop is a quadratic function of flow. As an illustrative example, the estimated pressure drop may refer to resistance Ri, as described above with reference to FIG. 1C.

Pressure controller 222 is configured to generate an instruction that causes pump 204 to generate the gas flow such that the ambient air is pressurized. The amount of pressure induced by pump 204 via instructions from pressure controller 222 is such that the output gas flow has a pressure corresponding to a sum of the prescribed pressure input to generator 110 plus an estimated amount of pressure drop determined to exist within system 100 (e.g., due to pressure loss via channels 104 and 106) multiplied by the flow rate measured by flow sensor 114. Therefore, the gas flow will be delivered to interface 130 from generator 110. Furthermore, the flow rate of the output gas flow will, in one embodiment, be held fixed by controller 160 in communication with flow controller 150 based on measured values obtained by sensors 112 and 114, and therefore pressure of the gas flow provided to the patient will also remain fixed (e.g., a pressure variance of less than 5%).

The pressure, in one embodiment, is measured at an outlet of generator 110 (e.g., where channel 104 interfaces with generator 110 and/or flow controller 150). The measured pressure is then compared with a prescribed pressure setting input to pressure command module 252 plus the estimated pressure drop detected by estimated pressure drop module 254. Estimated pressure drop module 254 is configured to measure a loss in the pressure based on the measured flow rate from sensor 114 and the gas characteristics (e.g., leaks, constrictions, etc.) of channel 104, humidifier 140, channel 106, and nasal interface 130. An error signal is derived as corresponding to a difference between the aggregate of the estimated pressure drop detected and the prescribed pressure setting input minus the output pressure detected by pressure sensor 112. This error signal is provided to pressure controller 222, which in turn is configured to cause pump 204 to modulate the output gas flow's pressure. For example, based on the error estimated, pressure controller 222 may cause a speed of a fan of pump 204 to speed up or slow down, a voltage sent to pump 204 to be modulated, a current provided to pump 204 to be modulated, and the like.

Returning to FIG. 2A, a proportional regulating valve 220 receives concentrated oxygen (or other gas, or mixtures of gases) from channel 226 and provides the concentrated oxygen to an $O_2$ flow sensor 216 to determine a flow rate of the input oxygen. The oxygen is then communicated to a mixer 206, which facilitates the blending of the received ambient air and the received concentrated oxygen. Mixer 206 then facilitates the mixed gas to be provided to a gas flow sensor 114 via a channel 230 fluidly in communication there between.

Gas flow sensor 114 is configured, in one embodiment, to determine a flow rate of the gas to be output by generator 110. Gas flow sensor 114 measures the flow rate of the gas and, in one embodiment, determines whether the flow rate of the gas is of the desired flow rate input to generator 110. Gas flow sensor 114 is configured to generate a digital signal that is then transmitted to a blending module 212 for controlling a flow rate of oxygen to produce the prescribed concentration of inhaled oxygen. In still further embodiments, as mentioned above, flow controller 150 is operable to adjust the gas flow output by generator 110 by increasing/decreasing resistance Re. In one embodiment, controller 160 may generate an output signal (e.g., a current) provided to flow controller 150 to cause resistance Re of flow controller 150 to change, thereby increasing or decreasing a value of Qe.

The concentration of oxygen, which is also referred to herein as "FiO2," for Fraction of inhaled oxygen, is prescribed by a clinician (e.g., a doctor, nurse, physician's assistant, respiratory therapist, etc.) at some value between 21% oxygen to 100% oxygen. For example, ambient air typically has approximately 21% oxygen. Therefore, if the oxygen in the gas to be output by ventilator is 21%, then this indicates that the amount of oxygen to be provided by source 202 is substantially zero. As another example, if the concentration of oxygen for the output gas is to be 100% oxygen, then this indicates that the amount of oxygen to be provided by source 202 is equivalent to the measured gas flow of sensor 114. In one embodiment, controller 160 is configured to generate an instruction indicating the oxygen concentration to be employed, which is then communicated to generator 110.

Gas flow sensor 114, in one embodiment, measures the flow of gas, and then employs Equation31 to determine a necessary amount of oxygen flow:

$$Q_{O_2} = \frac{FiO_2 - 0.21}{0.79} \cdot Q_{gas} \qquad \text{Equation 3.}$$

Here, $Q_{O_2}$ is the flow of pure oxygen through $O_2$ flow sensor 216, $FiO_2$ is a prescribed concentration of oxygen for the high-flow therapy to be delivered to the patient, 0.21 is a fraction of oxygen typically found in ambient air, 0.79 is a fraction of non-oxygen gas typically found in ambient air, and $Q_{gas}$ is the flow of gas through gas flow sensor 114.

$O_2$ flow Servo controller 218 uses the difference between the output of blending module 212 and the measurement of the gas flow from sensor 216 to regulate a proportional valve 220, thereby controlling an amount of oxygen gas entering mixer 206. The high pressure oxygen source 202 is connected to valve 220 through channel 226. These components and connection are typically at a high pressure (e.g., approximately 50 PSI). For example, gas source 202 may be an oxygen tank and components on the channels connected to the oxygen tank are reinforced for such high pressure, in accordance with various techniques known to those of ordinary skill in the art. The combination of sensor 216, controller 218, and valve 220 are configured to control the flow of pure oxygen to sensor 216 based on the measured gas flow at gas flow sensor 114 and the relationship provided by blending module 212 at frequencies above a highest component of a respiratory waveform. For example, a patient's respiratory mechanics typically prohibit any significant flow changes above 3 Hz and the controller can respond quickly enough such that the concentration of gas in all segments of the breathing circuit will be uniformly equal to the FiO2 setting that is prescribed.

In one embodiment, generator 110 includes one or more instance of processor(s) 240, memory 242, communications circuitry 244, and I/O interface(s) 246. In another embodiment, one or more instances of processor(s) 240, memory 242, communications circuitry 244, and/or I/O interface(s) 246 is/are located external to generator 110, and are in communication with generator 110 to control one or more functions of generator 110. For example, generator 110 may be in communication with controller 160. As another example, as described in greater detail below, one or more of processor(s) 240, memory 242, communications circuitry 244, and I/O interface(s) 246 may be located on one or more user devices.

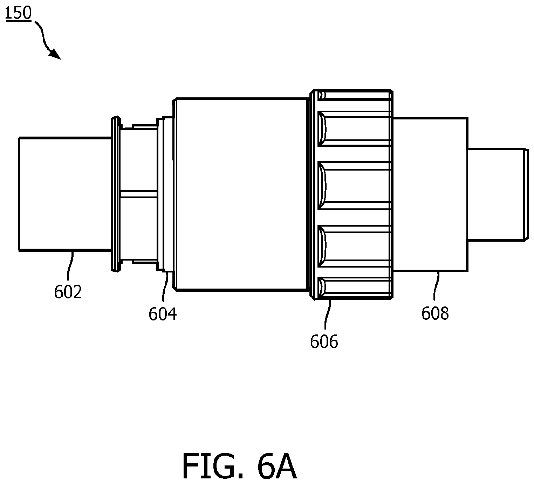

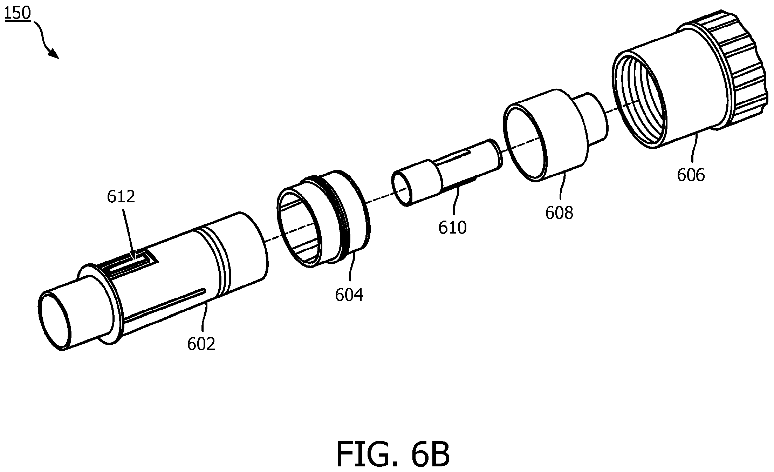

As mentioned previously, system 100 further includes, in one embodiment, flow controller 150. Flow controller 150 is capable of adjusting an amount of the gas flow output to the ambient environment to ensure that the pressure of the gas flow at patient interface 130 remains substantially constant. In one embodiment, flow controller 150 includes a manually adjustable component to control the output flow (e.g., Qe), as described by FIGS. 6A and 6B. Through, for example, a mechanical process (e.g. turning a threaded valve) the flow rate Qi of the gas flow is adjustable with some or no feedback from controller(s) 160. When there is feedback, the output gas flow to the patient can be adjusted according to the patient's comfort, a therapeutic efficacy, and/or adjustments to flow controller 150. For example, flow controller 150 is adjusted by control signals received from controller 160. For instance, as mentioned herein, controller 160 is operable to adjust an output setting of flow controller 150b based on output signals received by controller 160 from sensors 112 and/or 114. The output flow of gas exiting system 100, for instance, may be continuously displayed by I/O interface 246, and further by providing visual feedback for adjusting - in a manual process - the flow controller 150.

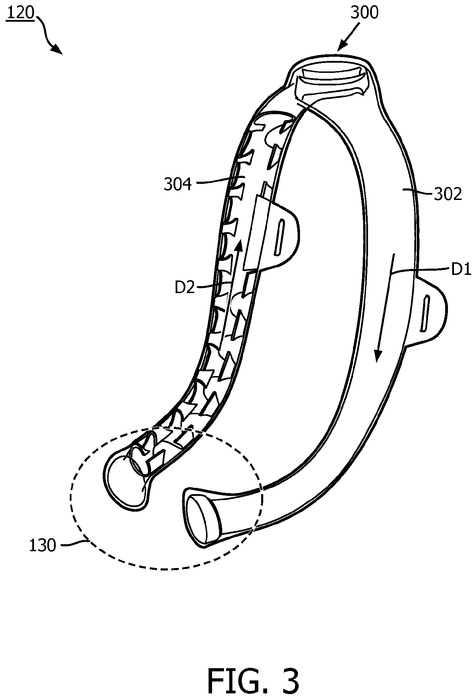

FIG. 3 is an illustrative diagram of patient interface 120 used within the exemplary system of FIG. 1, in accordance with various embodiments. Patient interface 120, in the illustrative embodiment, is configured to reside on patient 102 such that patient 102 is unencumbered and able to perform a vast number of daily functions such as speaking, eating, sleeping, and the like. Patient interface 120 is fluidly connected to one or more of channels 106 and 108 such that heated and humidified high-flow gas is capable of being received by patient interface 120 and provided to nasal interface 130 (not shown in FIG. 3).

In a non-limiting embodiment, the heated and humidified high-flow gas is received by patient interface 120 at a port 300. Port 300 includes, in one embodiment, a divider, capable of directing the flow of the heated and humidified high-flow gas to one of a first conduit 302 or a second conduit 304. Each of conduits 302 and 304 are designed such that they comfortable fit on a patient's head, and drape back behind, or substantially proximate to, a patient's ears. Conduits 302 and 304 are substantially tubelike structures having a substantially circular cross section that allows gas and/or fluid to freely move there through. In one embodiment, patient interface 120 is a substantially unitary structure such that port 300 and conduits 302 and 304 are integrally formed. For example, patient interface 120 may be formed from a low-weight, high flexibility plastic made via a molding process. Although not shown, a strap or other securing mechanism may also be connected to conduits 302 and/or 304 to assist in securing patient interface 120 to patient 102.

As mentioned above, the divider directed the heated and humidified high-flow gas into one of first conduit 302 or second conduit 304. For example, in the illustrative embodiment, the flow of the heated and humidified high-flow gas is directed by the divider from port 300 to first conduit 302. The heated and humidified high-flow gas is then able to flow through first conduit 302 in a first direction D1 towards an opening at an end of first conduit 302 opposite of port 300 whereby the heated and humidified high-flow gas will enter nasal interface 130. Nasal interface 130, although not seen within FIG. 3, is capable of outputting the heated and humidified high-flow gas to patient 102 such that a jetting effect is produced, while also providing a substantial seal to patient 102 thereby reducing an amount of leak at nasal interface 120.

Some or all of exhaled gas from patient 102, as well as some or all of the output heated and humidified high-flow gas, may then be received by second conduit 304. The received gas is then capable of traveling up second conduit 304 in a direction D2 opposite that of direction D1. The remaining gas is then capable of being vented to the ambient environment and/or returned to generator 110 or another portion of system 100.

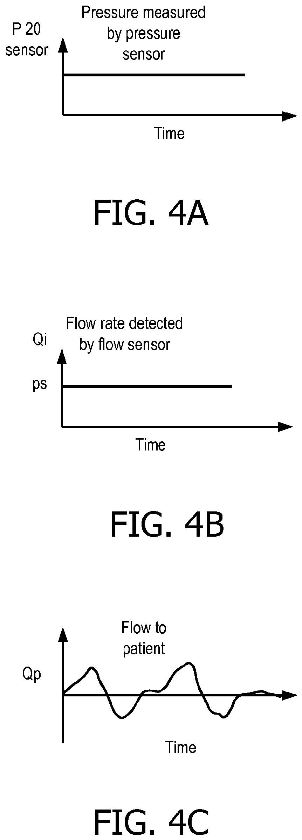

FIGS. 4A-E are illustrative graphs describing exemplary pressure measurements, flow rates, patient flow rates, current values, and breathing rates, in accordance with various embodiments. FIG. 4A is an illustrative graph of an amount of pressure measured by pressure sensor 112 within system 100. The pressure of the gas flow, as generated by generator 110, is configured to be substantially constant during the course of the therapy. As detailed above with reference to Equation 2, the measured pressure is based on the prescribed pressure (e.g., an amount of pressure for the gas flow a patient is to receive), a flow rate measured by flow sensor 114 (e.g., $Q_i$) and a resistance, or pressure drop, within system 100 (e.g., $R_i$). In one embodiment, controller 160 is configured to generate an instruction that causes, if necessary, generator 110 to increase or decrease a pressure of the output gas flow. For example, generator 110 may cause a fan to increase its speed or decrease its speed, thereby increasing or decreasing the pressure of the output gas flow, respectively.

FIG. 4B is an illustrative graph of a flow rate detected by flow sensor 114 within system 100. As seen from FIG. 4B, the measured flow rate (e.g., $Q_i$) is substantially constant as well. The reason being that, to ensure a constant pressure, as described above with reference to FIG. 4A, a substantially constant flow rate measured by flow sensor 114 is needed. As mention previously as well, in order to ensure a substantially constant flow rate, controller 160 is configured, in one embodiment, to module a current provided to flow controller 150 to thereby adjust an amount of gas flow being vented, to maintain the relationship between $Q_i$ and $Q_e$, $Q_p$, and $Q_l$, as described by Equation 1.

FIG. 4C is an illustrative graph of a gas flow to a patient. For instance, flow $Q_p$ corresponds to an amount of the gas flow that is inhaled by the patient. When the patient inhales, the flow rate increases as the patient is pulling in more gas. This is seen from the peaks of the graph within FIG. 4C. When the patient exhales, the flow rate decreases as the patient is expelling gas. This is seen from the valleys in the graph within FIG. 4C.

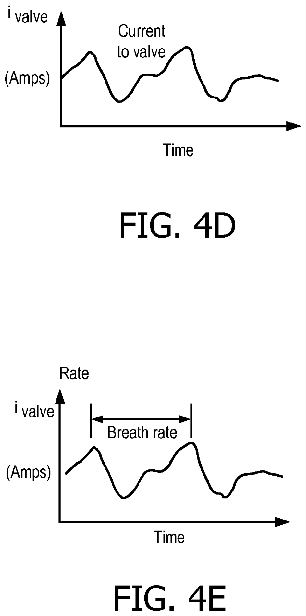

FIG. 4D is an illustrative graph of an amount of current provided to flow controller 150 within system 100. As an illustrative example, flow controller 150 may correspond to a valve, such as a throttle valve, capable of opening and closing to allow more gas to vent and less gas to vent, respectively. As seen from FIG. 4C, when the patient inhales, the amount of gas flow to the patient increases. Therefore, in response, the valve should begin to close, as reflected by the peak in current from FIG. 4D. When the patient exhales, the amount of gas flow to the patient decreases, and thus the valve should begin to open, as reflected by the valley in current from FIG. 4D. Therefore, various breathing characteristics (e.g., tidal volume, breath rate, etc.) of the patient are capable of being monitored by observing the change in current flow provided to the valve. Furthermore, although the illustrative embodiment shows that an increased current causes the valve to close, this is merely exemplary, and alternatively an increased current may cause the valve to open in some embodiments.

FIG. 4E is an illustrative graph describing how a breathing rate of a patient is determined by monitoring an amount of current provided to flow controller 150 (e.g., a valve). For instance, as mentioned before, in one example embodiment, when a current increases, thereby closing the valve, this may correspond to a patient inhaling. Therefore, by measuring an amount of time that elapses between consecutive peak current values may indicate a breathing rate of the patient. For example, if the amount of time between one current peak and a subsequent current peak is δt, then the breathing rate of an individual (e.g., breaths per minute) is capable of being determined. Furthermore, in one embodiment, multiple measurements for the amount of time between consecutive current peaks may be measured to determine an average amount of time, which in turn may be used to compute the breathing rate of the patient.

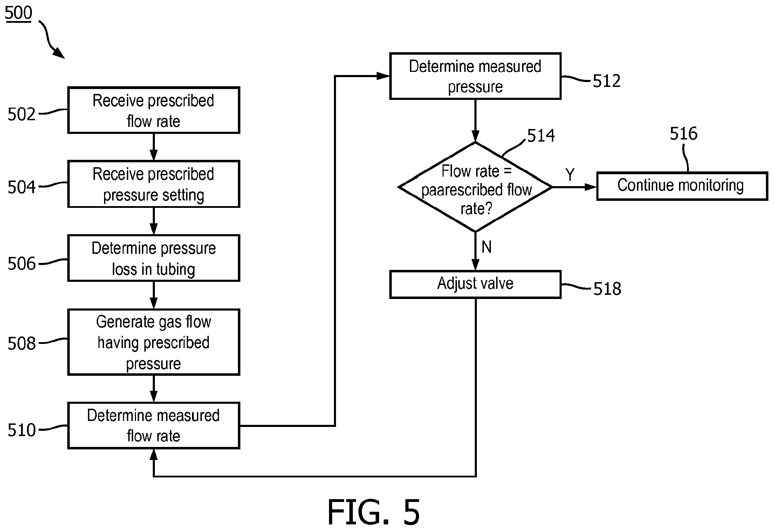

FIG. 5 is an illustrative flowchart of an exemplary process 500 for adjusting a valve, in accordance with various embodiments. In a non-limiting embodiment, process 500 begins at operation 502. At operation 502, a prescribed flow rate is received. For instance, a clinician may determine that a patient (e.g., patient 102) is to receive a breathing therapy having a flow rate of 60 lpm. At operation 504, a prescribed pressure setting is received. For instance, the clinician may determine that the patient is to receive the breathing therapy having a pressure of 10 cmH20. In one embodiment, the prescribed flow rate and the prescribed pressure are received at a substantially same time. For instance, a clinician may input the prescribed flow rate and the prescribed pressure into controller 160 using I/O interface 246.

At operation 506, an amount of pressure lost between the generator (e.g., generator 110) and the nasal interface (e.g., nasal interface 130) is determined. For instance, the pressure drop, or pressure loss, may be known to controller 160. As an illustrative example, the pressure loss may correspond to resistance Ri (e.g., 10 cmH20/lps). At operation 508, a gas flow is generated having the prescribed pressure. For instance, based on the known pressure loss and the prescribed pressure, controller 160 causes generator 110 to generate a gas flow to meet the desired pressure for the breathing therapy. As an illustrative example, if the pressure loss is 10 cmH20/lps, the flow rate is 1 lps, and the prescribed pressure is 10 cmH20, then controller 160 may generate an output signal to cause generator 110 to generate a gas flow having a pressure of 20 cmH20. By doing so, as described above by Equation 2, the pressure of the gas flow delivered to the patient will be the prescribed 10 cmH20.

At operation 510, a measure flow rate is determined. For instance, the flow rate is measured by flow sensor 114. In one embodiment, flow sensor 114 measures the flow rate of the gas flow and provides data representing the measured flow rate to controller 160. At operation 512, a measured pressure is determined. For instance, pressure sensor 112 measures the pressure of the gas flow, and provides data representing the measured pressure to controller 160.

At operation 514, a determination is made as to whether the measured flow rate equals the prescribed flow rate. If, at operation 514, it is determined that the measured flow rate differs from the prescribed flow rate, then process 500 proceeds to operation 518. For example, flow sensor 114 may determine that the measured flow rate Qi is 50 lpm, whereas the prescribed flow rate is 60 lpm. At operation 518, a valve is adjusted. For instance, a valve (e.g., flow controller 150) is adjusted to cause an amount of gas vented from the valve to increase or decrease to restore (or attempt to restore) the measured flow rate to the prescribed flow rate. As an illustrative example, if the measured flow rate is greater than the prescribed flow rate, then controller 160 may generate an output signal to flow controller 150 to cause flow controller 150 to close its valve, thereby balancing the sum of Qe, Ql, and Qp, to keep Qi constant. If, however, at operation 514, the measured flow rate is determined to match the prescribed flow rate (e.g., Qi is approximately the prescribed flow rate), then process 500 proceeds to operation 516. At operation 516, system 100 continues to monitor the flow rate and the pressure to ensure that the breathing therapy provided to the patient remains at the prescribed flow rate and prescribed pressure. For instance, after operation 516, process 500 may return to step 510, where the process loops.

Generally speaking, in high-flow nasal therapy, most healthy adults will inhale 12 L/min or less of gas flow. When a gas flow is prescribed at a high-flow rate, such as 40 L/min, to a patient, some of that 40 L/min will be inhaled by the patient, some will be leaked to the ambient environment, and some will remain. When a leak (e.g., between nasal interface 130 and the ambient environment) is excessive, flow controller 150 will close, in one embodiment. In this particular example, upon flow controller 150 closing, controller 160 identifies a difference between the measured flow rate and the prescribed flow rate. When the patient is breathing, flow controller 150 receives instructions to adjust an amount of gas flow vented in a systematic, and repetitive, manner to ensure constant, or substantially constant, pressure and flow rate to the patient. For example, the breathing pattern of a patient has a natural ebb and flow, that can be seen by changes to the amount of gas flow inhibited by flow controller 150. The patterned breathing detected by controller 160 (and in turn from the current provided to flow controller 150) indicate that the patient is receiving the breathing therapy, and also that the patient is breathing in a normal manner.

In one embodiment, one or more respiratory characteristics of a patient are monitored. For example, and without limitation, a tidal volume during inspiration and expiration may be monitored, a breathing rate may be monitored, a blood-oxygen concentration level may be monitored, and so on. For instance, the tidal volume during inspiration and/or expiration may be monitored by controller 160 based on the adjustments made to flow controller 150 (e.g., adjustments to the valve at operation 518).

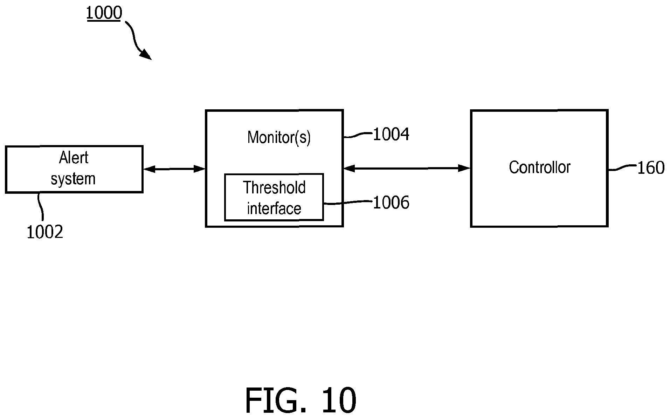

In one embodiment, one or more comparisons are performed by which at least one of the one or more respiratory characteristics are identified based on the various adjustments made to the valve, and which are then compared to a threshold value associated with that respiratory characteristic. For example, a breathing rate threshold may be input by a clinician into a threshold interface (e.g., threshold interface 1004 of FIG. 10). The breathing rate received by determined by controller 160 may then be compared to the breathing rate threshold to determine whether the measured breathing rate is less than the threshold, equal to the threshold, or greater than the threshold, and further may determine an extent, if any, of how different the measured breathing rate is in relation to the breathing rate threshold. For example, data representing the amount of current provided to flow controller 150 by controller 160 to restrict the amount of gas flow vented may be provided to monitoring system 1006 from controller 160, as described below with reference to FIG. 10. Monitoring system 1006 is configured, in the example embodiment, to check for abnormal situations (e.g., irregular breathing pattern, decreased/increased tidal volume, separation between the patient and nasal interface 130, etc.) associated with the patient. Monitoring system 1006, in one embodiment that includes patient information, such as data representing breathing patterns of the patient, tidal volumes of the patient, etc.) is stored in memory of monitoring system 1006 and used to identify when/if the patient's breathing is not normal.

A determination is capable of being made as to whether or not the threshold is met. For example, a determination is made as to whether the breathing rate measured is greater than a threshold value. If it is determined that the threshold has not been met, then the respiratory characteristics are continually measured. If, however, it is determined that the threshold has been met, then an alert is generated. Persons of ordinary skill in the art will recognize that the threshold being "met" may correspond to a particular measured characteristic value exceeding the threshold, exceeding the threshold by a particular amount, equaling the threshold, being less than the threshold, or being less than the threshold by a particular amount, and may depend on the particular characteristic being measured. For example, if the respiratory characteristic is blood oxygen level, then if the threshold is 98%, the measured blood oxygen level is said to be met if the measured blood oxygen level is less than the threshold. As another example, if the respiratory characteristic is breathing rate, then if the breathing rate threshold is 12 breaths per minute, the measured breathing rate is said to meet the threshold if the measured breath rate is less than 10 breaths per minute or more than 14 breaths per minute.

In one embodiment, monitoring system 1006 generates an alert to indicate that the threshold has been met, indicating an abnormal patient behavior is/was detected. The alert generated may correspond to audio data, text data, image data, or any other form of data capable of alerting one or more alert systems (e.g., alert system 1002). For example, the alert may correspond to an audible tone to be output by alert system 1002, which may correspond to a mobile device including a speaker that alerts a caregiver to check on the status of the patient. In one embodiment, the alert corresponds a text message, telephone call, email, or any other type of digital message to be rendered by a user device of a caregiver and/or clinician.

FIGS. 6A and 6B are illustrative diagrams of a side view and perspective view of an exemplary flow controller 150, in accordance with various embodiments. Flow controller 150, in the illustrative, non-limiting embodiment, connects to patient interface 120 and in particular, nasal interface 130. Alternatively or additionally, another instance of flow controller 150 is capable of connecting to generator 110. FIG. 6A presents an assembled flow controller, whereas FIG. 6B shows a perspective view of the various components of the flow controller. In one example embodiment, flow controller 150 corresponds to a throttle valve.

High-flow gas (e.g., air, air blended with concentrated oxygen, substantially pure oxygen, etc.) is capable of entering into controller 150 via first component 602. The received air is then directed via component 602 to a component 610, which is aligned with component 602 along a longitudinal axis. The flow is configured by controller 150 to return to a component 608 toward an exhaust port 612 integrally formed by component 610. Flow is capable of being adjusted by controller 150 via components 604 and 606. For instance, component 606 moves component 604 over exhaust port 612, thereby partially to fully obstructing the flow from venting to the ambient environment. In one embodiment, flow controller 150 is operated via an electric coil, such that a machine (e.g., processors 240) are capable of receiving an input of the prescribed flow, or flow adjustment, and causing controller 150 to open or close port 612. In one embodiment, a digital readout is available via I/O interface 246 to indicate the flow change as controller 150 is adjusted. Furthermore, a lock feature is capable of being included that fixed a position of component 604 about port 612 thereby fixing the ventilation amount of controller 150.

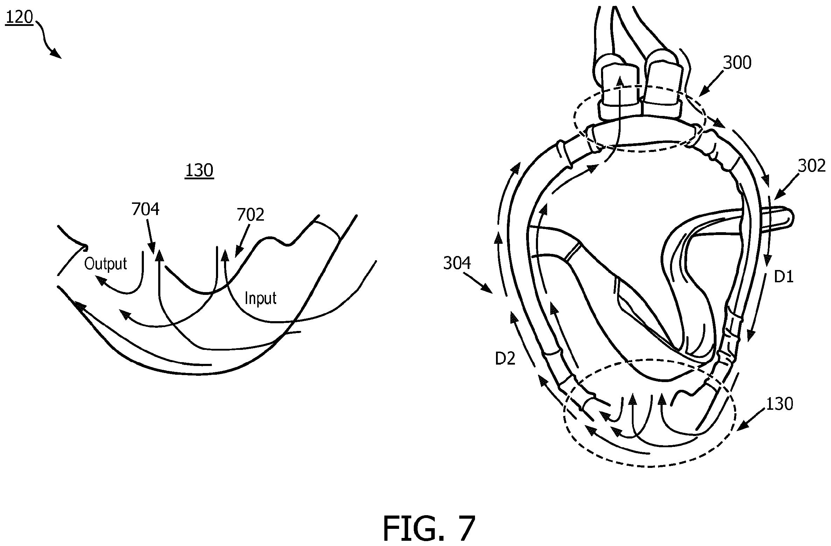

FIG. 7 is an illustrative diagram of a patient interface including a nasal interface, in accordance with various embodiments. In the non-limiting illustrative embodiment, patient interface 120 includes nasal interface 130, which has been presented in an expanded view. Nasal interface 130, as an illustrative embodiment, corresponds to a nasal pillow piece that fits within a space at the ends of conduits 302 and 304. As seen in FIG. 7, the heated and humidified high-flow gas is directed into conduit 302 via port 300. In the illustrated embodiment, port 300 is segmented into two separate interfaces - one that receives the heated and humidified high-flow gas and directs the heated and humidified high-flow gas to conduit 302, and one that receives expelled gas from nasal interface 130 and/or the patient and delivers the expelled gas to a vent for ventilation to the ambient environment. The pattern of the heated and humidified high-flow gas traveling down one side of the patient interface, through nasal interface 130, and back up conduit 304 allows for a pattern of bi-pass flow to be produced. Persons of ordinary skill in the art will recognize that, alternatively, a divider may be included within port 300 to direct the received heated and humidified high-flow gas to first conduit 302.

Nasal interface 130, in the non-limiting embodiment, includes a first opening 702 and a second opening 704.

Openings 702 and 704 are configured such that the heated and humidified high-flow gas received via conduit 302 is output to a patient's upper airway to provide respiratory therapy and a prescribed flow rate, oxygen concentration, and pressure setting. The configuration of openings 702 and 704 are such that a jetting effect is experienced, whereas the output gas is streamed across the nasal airway and inhaled by the patient. Furthermore, the configuration of openings 702 and 704 are such that at least a portion of the expelled gas from the patient, as well as at least a portion of the output gas, are able to be received by openings 702 and 704, and vented out via conduit 304.

Nasal interface 130 allows for a pressure at nasal interface 130 to be controlled using generator 110 and/or flow controller 150. For example, generator 110 may provide the gas flow at the prescribed pressure for the high-flow gas being delivered to the patient, while flow controller 150 may adjust an amount of the gas flow capable of exiting nasal interface 130 to maintain the pressure at nasal interface 130 and/or increase/decrease the pressure at nasal interface 130. In one embodiment, due to the seal created about the patient's nose via nasal interface 130, system 100 is capable of providing a particular pressure setting to a patient. For example, if the flow dialed in to generator 110 is 30 L/min, and the relationship between flow rate and pressure is for every 10 L/min of flow, there is 1 cmH2O pressure, then essentially 3 cmH2O pressure is provided by the 30 L/min flow. Therefore, even though there is airflow bypassing the nose of the patient, patient interface 130 is pressurized to the desired pressure.

Numerous advantageous features of high-flow nasal therapy are capable of being achieved. The first effect is the jetting of the high-flow gas into the airway thereby clearing out $CO_2$ gas from a previous exhalation. This further provides an added pressure to the airway, which facilitates the airways to the patient's lungs being "opened," thereby providing better ventilation, better $CO_2$ clearance, and better oxygenation of the blood.

Although only a single nasal interface 130 is shown within FIG. 7, in one embodiment, different nasal interfaces are capable of being used in conjunction with patient interface 120. Each nasal interface therefore has different shapes, configurations, and/or opening sizes/shapes, to alter a way in which the high-flow gas is provided to the patient, as well as a way that the expelled gas is received and vented. In one embodiment, a single nasal interface is included that has a variable control to adjust a size of an opening, or openings, a shape of the opening(s), and/or modify any other suitable parameters of the nasal interface. For instance, nasal interface 130 may include different configuration options capable of being used for different patients and/or therapies. For example, a first nasal interface having a first cannula configuration (e.g., one or more opening having a particular size and shape) may be used for one therapy, while a second nasal interface having a second cannula configuration (e.g., one or more openings having a different size and/or shape) may be used for another therapy.

In yet another embodiment, nasal interface 130 is variable such that a size and/or shape of an opening, or openings, of nasal interface 130 is/are capable of being adjusted. In this particular scenario, nasal interface 130 and/or patient interface 120 are coupled to one or more port area controls configured to adjust the one or more openings of nasal interface 130. For example, port area control(s) may correspond to a valve, switch, or digital interface, which may be employed to modify a size of an opening within nasal interface 130 with which the heated and humidified high-flow gas will exit nasal interface 130 and be provided to the patient.

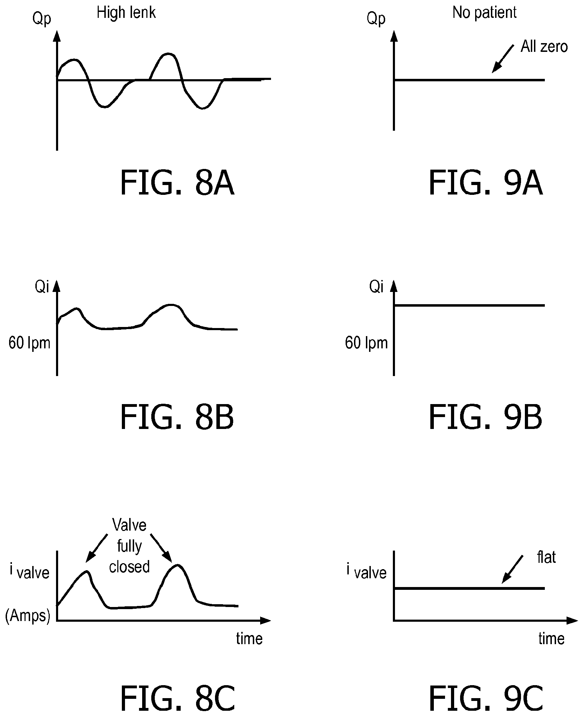

FIGS. 8A-C are an illustrative graphs of a detected high leak scenario, in accordance with various embodiments. FIG. 8A is an illustrative graph describing the gas flow to a patient in a high leak scenario (e.g., Ql > Qp). In this particular scenario, the flow to the patient (e.g., patient 102) may remain substantially similar in shape as that of FIG. 4C in that the patient may still be breathing. However, in this particular scenario, the magnitude of Qp is less than it would be in FIG. 4C.

FIG. 8B is an illustrative graph describing the flow rate measured by flow rate sensor 114. In this particular scenario, the flow rate measured is greater than the prescribed 60 lpm flow rate. In particular, the flow rate during inhalation further increases the measured flow rate to values much greater than the prescribed flow rate.

FIG. 8C is an illustrative graph describing a current flow to a valve during the high leak scenario depicted by FIGS. 8A and 8B. As seen from FIG. 8C, as the measured patient flow increases about the prescribed flow rate, the current provided to flow controller 150 to close flow controller also increases. In one embodiment, controller 160 is configured to determine whether the current exceeds a threshold value for a certain amount of time, and identifies the high leak scenario based on these criteria. For example, as controller 160 continues to try and close flow controller 150 at each inhalation of the patient, the current increases beyond the threshold value at particular temporal intervals consistent with a patient's breathing rate. Therefore, controller 160 may provide the current information to monitoring system 1006, as described below with reference to FIG. 10, which may determine that the threshold specified by threshold interface 1004 has been exceeded, and in turn generate an alert signal and send that alert signal to alert system 1002.

FIGS. 9A-C are illustrative graphs of a no patient detected scenario, in accordance with various embodiments. FIGS. 9A-C relate to various scenarios where a patient appears to not be connected to nasal interface 130. For instance, the patient may have removed nasal interface 130, nasal interface 130 may have broken, or a nasal outlet may be clogged. As seen in FIG. 9A, the patient flow in this particular scenario is substantially zero (e.g., 0 lpm). This is because there are no effects felt by system 100 due to inhalations/exhalations from the patient due to the patient being non-present or the nasal outlets being clogged.

FIG. 9B is an illustrative graph of the flow rate in the no patient detected scenario. As seen from FIG. 9B, the flow rate as measured by flow sensor 114 is substantially constant at the prescribed flow rate (e.g., 60 lpm). While this may appear to correspond to standard breathing characteristics (e.g., as seen from FIG. 4B), observing FIG 9C reveals additional information. In FIG. 9C, the current provided from controller 160 to flow controller 150 is constant. This indicates that there is no change to the current, which corresponds to a lack of patient breathing (e.g., FIG. 9A) being detected.

In one embodiment, monitoring system 1006 is configured to determine, based on the data representing the amount of current provided from controller 160 to flow controller 150, that there is no motion of flow controller 150 (e.g., no motion of the valve or resistance Re). Monitoring system 1006, in one embodiment, is configured to determine that if the amount of current remains constant for longer than a predefined amount of time, then this indicates a "no patient detected" scenario and, in turn, generates an alert and sends that alert to alert system 1002.

FIG. 10 is an illustrative diagram of an alert system and monitor in communication with a controller, such as that of FIGS 1A-C, in accordance with various embodiments. System 1000 further includes, in the illustrative embodiment, controller 160, which is in communication with one or more gas monitor 1004. Gas monitor(s) 1004 are further in communication with alert system 10002.

Monitor(s) 1004 are coupled to controller 160, in one embodiment, and allow the outputs of measured pressure and measure flow rate to be monitored. For instance, monitor(s) 1004 are capable of determining when a flow rate exceeds a threshold, a pressure exceeds a threshold, a current exceeds a threshold, or a leak is detected in excess of a threshold. In one embodiment, monitor(s) 1004 includes a threshold interface 1006 capable of receiving input thresholds from a patient and/or clinician, which are then used to determine when one or more breathing parameters of the patient (e.g., tidal flow, respiratory rate, blood oxygen level, etc.) are not within a normal or specified range for that patient. If an abnormal event is determined to be present by monitor(s) 1006, then monitor(s) 1006 is configured to generate an alert and send the alert to an alert system 1002.

Alert system 1002, in one embodiment, corresponds to a device such as, and without limitation, a mobile device, an alarm (e.g., audible alarm, visual alarm, etc.) to indicate that an abnormal event is occurring for system 1000. For example, if nasal interface 130 becomes disconnected to the patient, then controller 160 may determine that a current provided to flow controller 150 may remain constant, indicating that no breathing pattern is detected. As another example, if a high leak occurs, then the current being provided to flow controller 150 may exceed a threshold current value, indicating that an abnormal situation is occurring. In this particular scenario, monitor(s) 1006 determines that the leak detected at nasal interface 130 exceeds a threshold programed to threshold interface 1004, and generates and sends an alert to alert system 1002 to notify a caregiver to check on a status of the patient.

In the claims, any reference signs placed between parentheses shall not be construed as limiting the claim. The word "comprising" or "including" does not exclude the presence of elements or steps other than those listed in a claim. In a device claim enumerating several means, several of these means may be embodied by one and the same item of hardware. The word "a" or "an" preceding an element does not exclude the presence of a plurality of such elements. In any device claim enumerating several means, several of these means may be embodied by one and the same item of hardware. The mere fact that certain elements are recited in mutually different dependent claims does not indicate that these elements cannot be used in combination.

Although the description provided above provides detail for the purpose of illustration based on what is currently considered to be the most practical and preferred embodiments, it is to be understood that such detail is solely for that purpose and that the disclosure is not limited to the expressly disclosed embodiments, but, on the contrary, is intended to cover modifications and equivalent arrangements that are within the scope of the appended claims.

**Claims**

1. A nasal therapy system (100) comprising:

    a nasal interface (130);
    at least one flow sensor (114) configured to determine a flow rate of a gas flow to a patient via the nasal interface;
    at least one pressure sensor (112) configured to determine an amount of pressure of the gas flow output by generator (110);
    a valve (150) in communication with the nasal interface and operable to control an amount of the gas flow capable of being vented to an ambient environment; and
    a controller (160) operatively coupled to the at least one flow sensor, the at least one pressure sensor, and the valve, wherein the controller is configured to:

        determine a flow rate difference between a prescribed flow rate for a breathing therapy to be provided to the patient and the flow rate, and

cause an output setting of the valve to be adjusted to control the amount of the gas flow being provided to the nasal interface such that the amount of the gas flow being provided to the nasal interface is maintained at the prescribed flow rate and the prescribed pressure, wherein the output setting is determined based on the flow rate difference, **characterized in that**, the controller is further configured to:

determine a change in the flow rate by measuring an amount of current provided to the valve to cause the valve to close and/or open; and
determine, based on the amount of current and the change in the flow rate, one or more breathing characteristics associated with the patient.

2. The nasal therapy system of claim 1, wherein the nasal interface comprises:
at least one nasal outlet, a gas inlet, and a gas outlet, wherein at least a portion of the gas flow is output to a nasal airway of the patient via the at least one nasal outlet, the portion being related to the prescribed flow rate, the amount of the gas flow that is being provided to the nasal interface, and an additional amount of the gas flow leaked from the nasal interface.

3. The nasal therapy system of claim 1, further comprising:
a gas monitor in communication with the controller, the gas monitor being configured to:

generate an alert in response to determining that one or more breathing characteristics of the patient meet an alert condition, and
send the alert to at least one alert system.

4. The nasal therapy system of claim 1, further comprising:
a pump configured to generate the gas flow, wherein the controller is further configured to:
generate an output signal that causes the pump to increase or decrease an output pressure of the gas flow exiting the pump based on a difference between the amount of pressure of the gas flow and the prescribed pressure.


**Patentansprüche**

1. Nasentherapiesystem (100), umfassend:

eine Nasenschnittstelle (130);
mindestens einen Flusssensor (114), der konfiguriert ist, um eine Flussrate eines Gasflusses zu einem Patienten über die Nasenschnittstelle zu bestimmen;
mindestens einen Drucksensor (112), der konfiguriert ist, um eine Druckmenge des vom Generator (110) ausgegebenen Gasstroms zu bestimmen;
ein Ventil (150), das mit der Nasenschnittstelle in Kommunikation steht und betreibbar ist, um eine Menge des Gasflusses zu steuern, die in eine Umgebung entlüftet werden kann; und
eine Steuerung (160), die operativ mit dem mindestens einen Durchflusssensor, dem mindestens einen Drucksensor und dem Ventil gekoppelt ist, wobei die Steuerung konfiguriert ist zum:

Bestimmen einer Flussratendifferenz zwischen einer vorgeschriebenen Flussrate für eine dem Patienten bereitzustellende Atemtherapie und der Flussrate, und
Bewirken, dass eine Ausgabeeinstellung des Ventils angepasst wird, um die Menge des Gasflusses zu steuern, die an die Nasenschnittstelle geliefert wird, so dass die Menge des Gasflusses, die an die Nasenschnittstelle geliefert wird, auf der vorgeschriebenen Flussrate und dem vorgeschriebenen Druck gehalten wird, wobei die Ausgabeeinstellung basierend auf der Durchflussratendifferenz bestimmt wird, **dadurch gekennzeichnet, dass** die Steuerung ferner konfiguriert ist zum:

Bestimmen einer Änderung der Durchflussrate durch Messen einer Strommenge, die dem Ventil zugeführt wird,
um zu bewirken, dass sich das Ventil schließt und/oder öffnet; und
basierend auf der Strommenge und der Änderung der Flussrate, Bestimmen einer oder mehrerer dem Patienten zugeordnete Atmungseigenschaften.

2. Nasentherapiesystem nach Anspruch 1, wobei die Nasenschnittstelle umfasst:

mindestens einen Nasenauslass, einen Gaseinlass und einen Gasauslass, wobei mindestens ein Teil des Gasflusses über den mindestens einen Nasenauslass an einen nasalen Atemweg des Patienten ausgegeben wird, wobei der Teil mit der vorgeschriebenen Flussrate, der Menge des Gasflusses, die der Nasenschnittstelle zugeführt wird, und einer zusätzlichen Menge des aus der Nasenschnittstelle austretenden Gasflusses in Beziehung steht.

**3.** Nasentherapiesystem nach Anspruch 1, ferner umfassend:
ein Gasmonitor in Kommunikation mit der Steuerung, wobei der Gasmonitor konfiguriert ist zum:

Erzeugen eines Alarms als Reaktion auf die Bestimmung, dass eine oder mehrere Atmungseigenschaften des Patienten einen Alarmzustand erfüllen, und
Senden des Alarms an mindestens ein Alarmsystem.

**4.** Nasentherapiesystem nach Anspruch 1, ferner umfassend:
eine Pumpe, die konfiguriert ist, um den Gasfluss zu erzeugen, wobei die Steuerung ferner konfiguriert ist zum:
Erzeugen eines Ausgangssignals, das bewirkt, dass die Pumpe einen Ausgangsdruck des aus der Pumpe austretenden Gasstroms auf der Grundlage einer Differenz zwischen der Druckmenge des Gasstroms und dem vorgeschriebenen Druck erhöht oder verringert.

**Revendications**

**1.** Système de thérapie nasale (100) comprenant:

une interface nasale (130);
au moins un capteur de flux (114) configuré pour déterminer un débit d'un flux de gaz vers un patient via l'interface nasale;
au moins un capteur de pression (112) configuré pour déterminer une quantité de pression du flux de gaz délivré par le générateur (110);
une vanne (150) en communication avec l'interface nasale et utilisable pour commander une quantité du flux de gaz susceptible d'être évacué vers un environnement ambiant; et
un dispositif de commande (160) couplé de manière opérationnelle à l'au moins un capteur de flux, à l'au moins un capteur de pression, et à la vanne, où le dispositif de commande est configuré pour:

déterminer une différence de débit entre un débit prescrit pour une thérapie respiratoire à fournir au patient et le débit, et
faire en sorte qu'un réglage de sortie de la vanne soit ajusté pour commander la quantité du flux de gaz fourni à l'interface nasale de sorte que la quantité du flux de gaz fourni à l'interface nasale soit maintenue au débit prescrit et à la pression prescrite, où le réglage de sortie est déterminé sur la base de la différence de débit, **caractérisé en ce que** le dispositif de commande est en outre configuré pour:

déterminer un changement du débit en mesurant une quantité de courant fournie à la vanne pour amener la vanne à se fermer et/ou s'ouvrir; et
déterminer, sur la base de la quantité de courant et du changement du débit, une ou plusieurs caractéristiques respiratoires associées au patient.

**2.** Système de thérapie nasale selon la revendication 1, dans lequel l'interface nasale comprend:
au moins une sortie nasale, une entrée de gaz, et une sortie de gaz, où au moins une partie du flux de gaz est délivrée vers une voie nasale du patient via l'au moins une sortie nasale, la partie étant liée au débit prescrit, à la quantité du flux de gaz qui est fourni à l'interface nasale, et à une quantité supplémentaire du flux de gaz qui fuit de l'interface nasale.

**3.** Système de thérapie nasale selon la revendication 1, comprenant en outre:
un dispositif de surveillance du gaz en communication avec le dispositif de commande, le dispositif de surveillance du gaz étant configuré pour:

générer une alerte en réponse à la détermination qu'une ou plusieurs caractéristiques respiratoires du patient répondent à une condition d'alerte, et
envoyer l'alerte à au moins un système d'alerte.

**4.** Système de thérapie nasale selon la revendication 1, comprenant en outre:
une pompe configurée pour générer le flux de gaz, où le dispositif de commande est en outre configuré pour:
générer un signal de sortie qui amène la pompe à augmenter ou diminuer une pression de sortie du flux de gaz sortant de la pompe sur la base d'une différence entre la quantité de pression du flux de gaz et la pression prescrite.

FIG. 1A

FIG. 1B

FIG. 1C

FIG. 2A

EP 3 731 912 B1

FIG. 2B

FIG. 3

Pressure measured
by pressure
sensor

P 20
sensor

Time

## FIG. 4A

Flow rate detected
by flow sensor

Qi

ps

Time

## FIG. 4B

Flow to
patient

Qp

Time

## FIG. 4C

i valve

(Amps)

Current
to valve

Time

# FIG. 4D

Rate

i valve

(Amps)

Breath rate

Time

# FIG. 4E

EP 3 731 912 B1

<u>500</u>

```
        ┌──────────────────┐                                    ┌──────────────────┐
  502 ──│ Receive prescribed│                                    │ Determine measured│─── 512
        │     flow rate     │                          ┌────────▶│     pressure      │
        └──────────────────┘                          │         └──────────────────┘
                 │                                     │                  │
                 ▼                                     │                  ▼
        ┌──────────────────┐                          │              ╱  514  ╲
  504 ──│ Receive prescribed│                         │             ╱Flow rate =╲        ┌─────────────────────┐
        │  pressure setting │                         │            ╱ paarescribed ╲  Y   │  Continue monitoring │─── 516
        └──────────────────┘                          │            ╲   flow      ╱─────▶│                     │
                 │                                     │             ╲  rate?   ╱         └─────────────────────┘
                 ▼                                     │              ╲       ╱
        ┌──────────────────┐                          │                 │ N
  506 ──│ Determine pressure│                          │                 ▼
        │   loss in tubing  │                          │         ┌──────────────────┐
        └──────────────────┘                          │         │   Adjust valve   │─── 518
                 │                                     │         └──────────────────┘
                 ▼                                     │                  │
        ┌──────────────────┐                          │                  │
  508 ──│  Generate gas flow│                          │                  │
        │ having prescribed │                          │                  │
        │     pressure      │                          │                  │
        └──────────────────┘                          │                  │
                 │                                     │                  │
                 ▼                                     │                  │
        ┌──────────────────┐                          │                  │
  510 ──│ Determine measured│──────────────────────────┘                  │
        │     flow rate     │                                             │
        └──────────────────┘                                             │
                 ▲                                                        │
                 └────────────────────────────────────────────────────────┘
```

# FIG. 5

150

FIG. 6A

EP 3 731 912 B1

FIG. 6B

FIG. 7

Qp High lenk

FIG. 8A

Qi

60 lpm

FIG. 8B

i valve

(Amps)

Valve
fully
closed

time

FIG. 8C

Qp No patient

All zero

FIG. 9A

Qi

60 lpm

FIG. 9B

i valve

flat

time

FIG. 9C

EP 3 731 912 B1

1000

Alert
system

1002

Monitor(s) ─1004

Threshold
interface ─1006

Controllor ─160

FIG. 10

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- EP 0691134 A
- WO 0228460 A
- WO 2010076712 A
- WO 2011141845 A
- US 5865173 A
- WO 2011145014 A